(19) Europäisches Patentamt
European Patent Office
Office européen des brevets

(11) Numéro de publication : **0 290 313 B1**

# (12) FASCICULE DE BREVET EUROPEEN

(45) Date de publication du fascicule du brevet : **04.03.92 Bulletin 92/10**

(51) Int. Cl.$^5$ : **C07C 273/10,** C07C 243/04

(21) Numéro de dépôt : **88400999.4**

(22) Date de dépôt : **22.04.88**

## (54) Nouveau procédé d'obtention de nitrosocarbamates activés.

(30) Priorité : **22.04.87 FR 8705708**

(43) Date de publication de la demande : **09.11.88 Bulletin 88/45**

(45) Mention de la délivrance du brevet : **04.03.92 Bulletin 92/10**

(84) Etats contractants désignés : **AT BE CH DE ES FR GB GR IT LI LU NL SE**

(56) Documents cités :
**FR-A- 2 487 343**
**CHEMICAL ABSTRACTS, vol. 68, no. 23, 3 juin 1968, page 10099, résumé no. 104713e, Columbus, Ohio, US**
**JOURNAL MED. CHEM., vol. 25, no. 2, 1982, pages 178-182, American Chemical Society, Washington, US; J. MARTINEZ et al.: "Activated N-nitrosocarbamates for regioselective synthesis of N-nitrosoureas"**

(56) Documents cités :
**CHEMICAL ABSTRACTS, vol. 67, no. 9, 28 août 1967, page 4072, résumé no. 43489s, Columbus, Ohio, US; Y. AHMAD et al.: "Acylarylnitrosamine reaction. I. Use of nitrosylsulfuric acid in the preparation of unsymmetrical biaryls"**

(73) Titulaire : **SANOFI**
**40, Avenue George V**
**F-75008 Paris (FR)**

(72) Inventeur : **Roger, Pierre**
**6, rue Paul Valéry**
**F-78423 Montigny-les-Bretonneux (FR)**
Inventeur : **Fournier, Jean-Paul**
**7, rue La Condamine**
**F-75017 Paris (FR)**
Inventeur : **Leroy, Rolande**
**74, rue des Cévennes**
**F-75015 Paris (FR)**

(74) Mandataire : **Moncheny, Michel et al**
**c/o Cabinet Lavoix 2 Place d'Estienne d'Orves**
**F-75441 Paris Cedex 09 (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention a pour objet un nouveau procédé d'obtention de nitrosocarbamates activés permettant, d'une part, d'éviter l'utilisation d'isocyanates et permettant, d'autre part, la nitrosation dans des conditions avantageuses, notamment relativement au rendement et à la pureté des nitrosocarbamates obtenus.

Le procédé de l'invention s'applique avec avantage notamment à la préparation de nitrosourées, sans avoir recours à l'utilisation d'un isocyanate.

Jusqu'à ce jour, les isocyanates servent à la préparation de nitrosourées et, pour ce faire, sont mis à réagir avec une amine primaire ou secondaire, ce qui conduit à l'obtention de la fonction

$$-N-\underset{\underset{O}{\|}}{C}-NH,$$

laquelle est ensuite éventuellement nitrosée, par exemple à l'aide d'un nitrite de métal alcalin tel que le nitrite de sodium.

On utilise également les isocyanates pour préparer les nitrosourées dans un schéma réactionnel qui consiste à faire réagir un isocyanate, tel que l'isocyanate de méthyle, l'isocyanate de chloro-2 éthyle ou le cyclohexylisocyanate, sur un phenol activé, tel que le trichlorophenol-2,-4,-5, le pentachlorophenol ou le penta fluorophenol (cf. brevet français n° 80 16453, J. Med. Chem. vol 25, n° 2, 1982, 178-182), ce qui conduit à l'obtention d'urées lesquelles sont ensuite transformées en nitrosourées par le chlorure de nitrosyle.

Or, les isocyanates sont des produits relativement toxiques, qui compte tenu de certaines réglementations doivent être fabriqués sur place et utilisés in situ.

Pour éviter d'utiliser des isocyanates dans la préparation des dérivés de nitrosourées, on a également proposé (cf. Madelmont et al, Journal of Labelled Compounds and radiopharmaceuticals - vol. XXII n° 8, p. 851-862), le N-(chloro-2 éthyl)-carbamate paranitrophenyle $^{14}C$, obtenu par réaction du chloroformiate de paranitrophényle sur la chloro-2 éthylamine $^{14}C$, la nitrosation étant ensuite effectuée par le chlorure de nitrosyle en vue de la préparation d'un produit marqué. Cependant, l'utilisation du N-(chloro-2 éthyl))-carbamate de paranitrophenyle présente l'inconvénient de ne donner accès qu'à certaines nitrosourées et dans des conditions réactionnelles peu favorables à la séparation des produits de réaction (cf brevet français n° 2 487 343).

On a également décrit la préparation de N-alkyl carbamates en faisant réagir un halogénoformiate avec une amine primaire. Dans le brevet français n° 2089433, on fait réagir un halogénoformiate portant un groupe fluorométhyle avec des amines de formule HNRR' ; dans le résumé des Chem. Abstract 68 n° 23, 3 juin 1968, p. 10099, on traite avec un ester de 2,4,6 trichlorophényl d'acide chlorocarbonique avec une alkylamine ou une cycloalkyle amine ; dans le brevet n° 10020971, on traite un halogénoformiate avec une amine primaire $RNH_2$ dans laquelle R est un noyau phényl ou un noyau phényl substitué.

Toutefois, aucun de ces documents antérieurs ne décrit ni ne suggère la nitrosation ultérieure des carbamates obtenus ou leur utilisation pour la préparation de nitrosourées.

On a également décrit la nitrosation de N-alkyl carbamate par divers agents de nitrosation, en particulier $N_2O_4$, $N_2O_3$, NOBr, NOCl, nitrites alcoyl, ou acide (chlorhydrique ou sulfurique) et le nitrite de sodium. Dans ce dernier cas, il peut y avoir des réactions parallèles, les rendements ne sont pas toujours satisfaisants et le produit obtenu est difficile à purifier (brevet français n° 73625 et brevet US n° 3345256). Aucun de ces documents ne décrit la nitrosation directe de carbamates à l'aide d'acide nitrosylsulfurique.

L'un des aspects de l'invention est de proposer un nouveau procédé de préparation de nitrosocarbamates évitant l'utilisation d'isocyanates.

L'un des autres aspects de l'invention est de proposer un nouveau procédé de préparation de nitrosourées, d'une part, évitant l'utilisation d'isocyanates et, d'autre part, permettant la nitrosation dans de bonnes conditions.

Ces différents aspects de l'invention sont obtenus, en mettant en oeuvre un procédé qui comporte la combinaison des deux étapes suivantes : la première étape consiste, à la place de l'isocyanate normalement utilisé, à faire réagir une amine primaire avec un halogénoformiate, notamment un chloroformiate, pour obtenir un carbamate activé et la deuxième étape consiste à faire réagir directement de l'acide nitrosylsulfurique sur le carbamate activé obtenu à l'issue de la première étape pour nitroser ledit carbamate activé et obtenir un nitrosocarbamate.

Le procédé selon l'invention d'obtention des composés de formule I

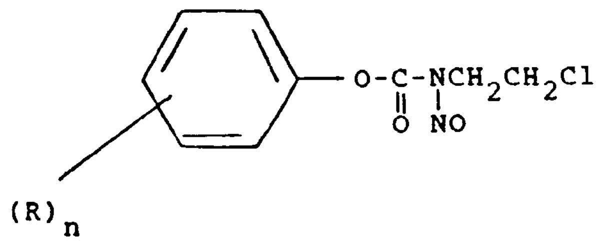

I

dans laquelle

– n vaut 2 à 5 et R représente Cl, Br ou F est caractérisé en ce que l'on fait réagir, dans une première étape, un halogénoformiate de formule II :

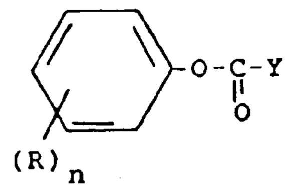

II

dans laquelle R et n ont les significations indiquées ci-dessus, et Y représente un halogène, notamment le chlore, sur l'amine $NH_2CH_2CH_2Cl$, pour obtenir le composé de formule Ibis

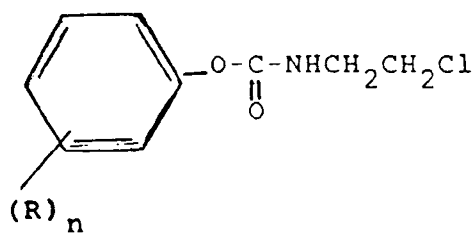

Ibis

dans laquelle R et n ont les significations indiquées ci-dessus,

– et en ce que, dans une deuxième étape, l'on traite directement le composé Ibis ci-dessus mentionné par l'acide nitrosylsulfurique.

Selon un autre mode de réalisation préféré du procédé de l'invention, l'halogénoformiate utilisé est un chloroformiate de formule IIbis suivante

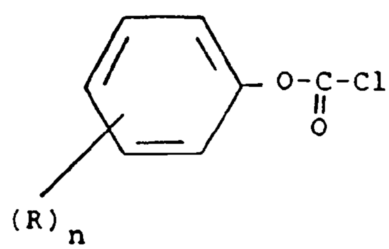

IIbis

dans laquelle R et n ont les significations indiquées ci-dessus.

Selon un mode de réalisation avantageux du procédé de l'invention, on utilise comme chloroformiate le pentachlorophenylchloroformiate ou le pentafluorophenylchloroformiate.

Selon un autre mode de réalisation du procédé de l'invention, l'halogénoformiate utilisé a comme formule

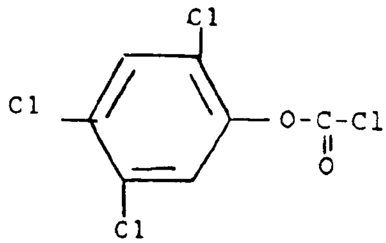

III

ce qui conduit, dans une première étape, après condensation de cet halogénoformiate sur $NH_2CH_2CH_2Cl$, au composé de formule IV

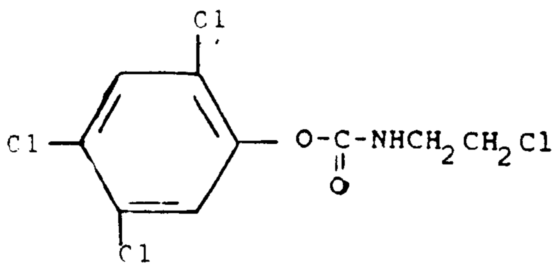

IV

lequel, dans une deuxième étape, est traité directement avec l'acide nitrosylsulfurique pour donner le composé de formule V :

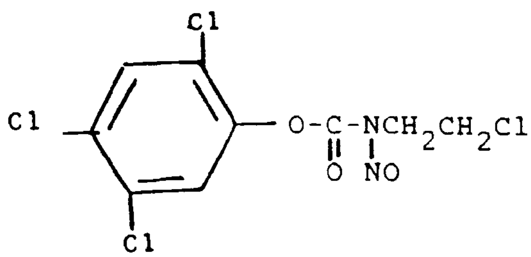

V

Parmi les halogénoformiates, notamment les chloroformiates utilisés dans le procédé de l'invention, le trichloro-2,4,5 phenylchloroformiate peut être obtenu dans le commerce, ou peut être fabriqué selon le procédé décrit dans Broadbent W.J. Chem. Soc. C, 1967, t. 24, pp. 2632-2636.

Les chloroformiates sont obtenus en faisant réagir le phénol comportant les substitutions appropriés avec le phosgène.

Le procédé de l'invention conduit à la préparation de nitrosocarbamates utiles à la préparation de nitrosourées, par condensation directe d'un nitrosocarbamate sur le groupe amine d'un sucre à partir duquel on veut former la nitrosourée correspondante.

L'invention sera mieux comprise à la lecture des exemples ci-après, qui servent à illustrer le procédé de l'invention sans être limitatifs.

Exemple 1 : Préparation d'un réactif utilisé dans le procédé de l'invention : le N-(chloro-2 éthyl)-N-nitrosocarbamate de trichloro-2,4,5 phényle.

Cette préparation se fait en deux étapes :

1) Préparation du N-(choro-2 éthyl)-carbamate de trichloro-2, 4, 5 phényle

Le schéma réactionnel est le suivant :

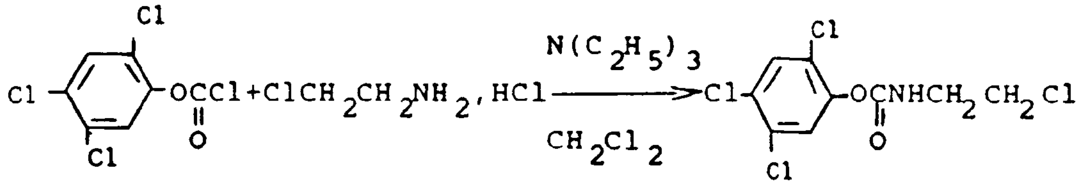


| | | |
|---|---|---|
| Réactifs : | trichloro-2,4,5 phénylchloroformiate | 10,4 g |
| | chlorohydrate de chloro-2 éthylamine | 4,65 g |
| | triéthylamine redistillée | 10 ml |
| | chlorure de méthylène anhydre | 200 ml |

A une solution de trichloro-2,4,5 phénylchloroformiate (10,4 g, 0,04 mole) dans du chlorure de méthylène anhydre (100 ml), est ajoutée, sous agitation à 0°C, et goutte à goutte, une solution de chlorhydrate de chloro-2 éthylamine (4,65 g, 0,04 mole) et de triéthylamine (10 ml) dans 100 ml de chlorure de méthylène anhydre.

Après 18 heures d'agitation à température ambiante, le milieu réactionnel est lavé à plusieurs reprises avec le minimum d'eau jusqu'à absence d'ion chlorure, la phase organique résultante est séchée sur sulfate de sodium puis évaporée à sec sous vide.

On isole le N-(chloro-2 éthyl)-carbamate de trichloro-2, 4, 5 phényle (11,1 g, 91 %) sous forme de cristaux blanc-gris.

F : 106°-107°C

CCM : éther de pétrole (40-65°C)-éther éthylique (2:1, v/v) ; Révélation iode.

R. f. : 0,4.

IR (KBr) : 3350 $cm^{-1}$ (NH), 1720 $cm^{-1}$ (carbonyle)

Spectre RMN (chloroforme-d) : 3,54-3,73 (m, $CH_2CH_2Cl$), 5,68 (s, NH), 7,36 et 7,53 (H aromatiques).

La pureté du trichloro-2,4,5 phénylchloroformiate doit être contrôlée avant sa mise en oeuvre (PF>60°C, Litt.F. : 65°, CCM Hexane-éther éthylique (2:1 v/v), Révélation UV monotâche Rf = 0,5).

Pour obtenir les normes précisées ci-dessus, une recristallisation dans l'hexane peut être dans certains cas nécessaire à partir du produit commercial.

Le spectre RMN du N-(chloro-2 éthyl)-carbamate de trichloro-2, 4, 5 phényle (100MHz) est représenté à la figure 1.

2) Préparation du N-(choro-2 éthyl)-N-nitrosocarbamate de trichloro-2, 4, 5 phényle

Le schéma réactionnel est le suivant :

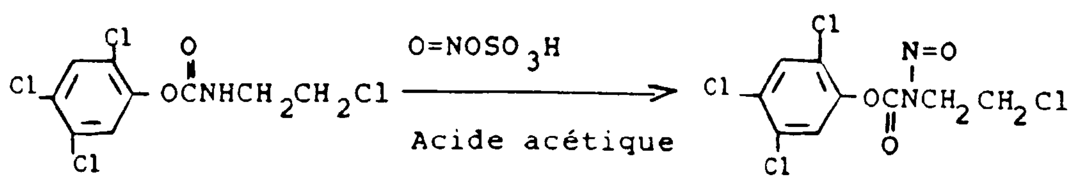


| | | |
|---|---|---|
| Réactifs : | N-(chloro-2 éthyl)-carbamate de trichloro-2, 4, 5 phényle | 12 g |
| | acide nitrosylsulfurique | 12,5 g |
| | acide acétique anhydre | 100 ml |

A une solution de N-(chloro-2 éthyl)-carbamate de trichloro-2, 4, 5 phényle (12 g, 0,04 mole) dans de l'acide acétique anhydre, est ajouté, à froid et par petites portions, de l'acide nitrosylsulfurique (12,5 g, 0,1 mole).

Après quatre heures d'agitation à 25°C, le milieu réactionnel est versé sur de l'eau (1 600 ml) puis extrait à l'éther éthylique, ; la phase organique est lavée à l'eau jusqu'à neutralité, séchée sur sulfate de sodium et filtrée. Le résidu obtenu par évaporation est repris par de l'éther de pétrole (40°C). Après 18 h à 0°C, les cristaux

formés sont essorés pour donner 11 (10,5 g, 80 %).

F : 62°C

CCM : Ether de pétrole (40-65°C)-éther éthylique (2:1, v/v) ; révélation : réactif de Griess.

R.f. : 0,8

IR (KBr) : 1770 $cm^{-1}$ (carbonyle), 1500 et 1480 $cm^{-1}$ (nitroso)

Spectre RMN (chloroforme-d) : 3,56 (t, $CH_2$-N, J=J'=6 Hz), 4,19 (t, $CH_2$-Cl, J=J'=6 Hz), 7,54 et 7,64 (s, H aromatiques).

Le spectre RMN du N-(chloro-2 éthyl)-carbamate de trichloro-2, 4, 5 phényle (100MHz) est représenté à la figure 2.

Exemple 2 :

Préparation du [(chloro-2 éthyl)-3 nitroso-3 ureido]-3 tridésoxy-2,3,6 β-L-arabino-hexopyrannoside de méthyle (IC 1615)

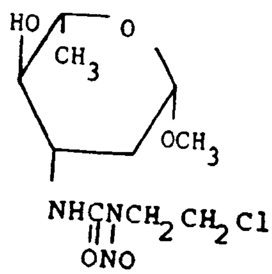

(IC 1615)

a) Préparation de l'azido-3 tridésoxy-2,3,6 β-L-arabino-hexopyrannoside de méthyle

3 g. (0,016 mole) d'azido-3 tridésoxy-2,3,6,α-L-arabino-hexopyrannoside de méthyle et 1 g. d'acide tosylique dans 50 ml de méthanol anhydre sont agités 48 heures à température ambiante.

Le milieu réactionnel est évaporé à sec sous vide. Le résidu est repris dans du chlorure de méthylène. Après deux lavages avec de l'eau, la phase organique séchée sur sulfate de sodium puis évaporée à sec sous vide est chromatographiée sur colonne de silice (éluant Hexane-Acetate d'éthyle 5:1)

Analyse : $C_7H_{13}N_3O_3$ = 187,20

F : 72°-73° (hexane)

$[\alpha]_D$ = + 63,5° (c 0,8 $CHCl_3$)

IR (Nujol) 3370 $cm^{-1}$ (azide)

b) Préparation de l'amino-3 tridésoxy-2,3,6 β-L-arabino-hexopyrannoside de méthyle

Ce composé est préparé en placant une solution de 5 g d'azido-3 tridésoxy-2,3,6 β-L-arabino-hexopyrannoside de méthyle dans 10 ml de méthanol, et enagitant 12 heures sous atmosphère d'hydrogène en présence de triethylamine (1 ml) et de charbon palladié à 10 % (1 g). Le catalyseur est éliminé par filtration.

Analyse : $C_7H_{15}NO_3$ = 161,20 Rdt. = 85 %

F : 136°

$[\alpha]_D$: + 75,8° (c 0,5 $CHCl_3$)

c) Préparation du [(chloro-2 éthyl)-3 nitroso-3 uréido]-3 tridésoxy-2,3,6 β-L-arabino-hexopyrannoside de méthyle

L'amino-3 tridésoxy-2,3,6 β-L-arabino-hexopyrannoside de méthyle est mis en solution dans du DMF, puis on ajoute du N-(chloro-2-éthyl)-N-nitrosocarbamate de trichloro-2,4,5 phényle, selon le procédé de l'invention.

Après 4 heures d'agitation à 0°C, le milieu réactionnel est évaporé. Le composé obtenu (IC 1615) est un produit nouveau et ses caractéristiques sont les suivantes :

Analyse : $C_{10}H_{18}ClN_3O_5$ = 295,7
F : 109°-110°
$[\alpha]_D$ = + 22,8° (c 0,5 % $CHCl_3$)

Exemple 3 :

Préparation du [(chloro-2 éthyl)-3 nitroso-3 uréido]-3 tridésoxy-2,3,6 β-D-arabino-hexopyrannoside de méthyle (IC. 1625)

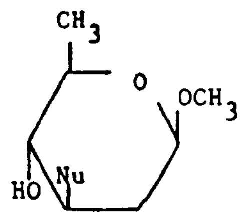

IC 1625

Nu = 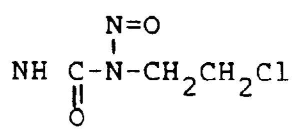

Ce composé est obtenu à partir d'amino-3 tridésoxy-2,3,6 β-D-arabino-hexopyrannoside de méthyle en utilisant le N-(chloro-2-éthyl)-N-nitroso-carbamate de trichloro-2,4,5-phényle selon le procédé de l'invention comme il a été décrit à propos de la préparation du composé IC 1615.

Le composé obtenu IC 1625 est nouveau.

Les caractéristiques du composé obtenu sont les suivantes :

Analyse : $C_{10}H_{18}ClN_3O_5$ = 295,7
F : 103°-105°
$[\alpha]_D$ = - 25,0° (c 0,3 % $CHCl_3$)

Exemple 4 :

Préparation du [(chloro-2 éthyl)-3 nitroso-3 uréido]-3 didésoxy-2,3 β-D-arabino-hexopyrannoside de méthyle (IC. 1673)

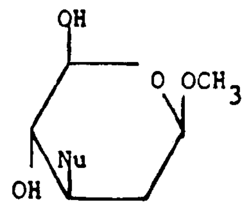

IC 1673

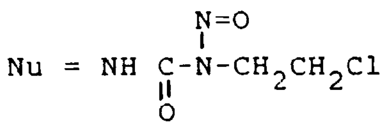

a) préparation de l'azido-3 didésoxy-2,3 β-D-arabino-hexopyrannoside de méthyle

19,2 g. (0,095 mole) d'azido-3 didésoxy-2,3 α-D-hexopyrannoside de méthyle préparé selon la demande de brevet européen n° 84 401743 et 1 g d'acide tosylique dans 200 ml de méthanol sont agités pendant 48 heures à température ordinaire. Le résidu obtenu après évaporation sous vide est repris dans 60 ml de pyridine anhydre.

On ajoute alors en maintenant la température à 15°, goutte à goutte, 30 g d'anhydride acétique. Après 16 heures sous agitation, on évapore à sec le milieu réactionnel. Le résidu est repris par du chlorure de méthylène ; la phase organique est lavée avec une solution d'HCl 2N, puis avec de l'eau, et enfin avec une solution de bicarbonate de sodium. La phase organique séchée sur sulfate de sodium est évaporée sous vide donne après chromatographie sur silice (éluant hexane 4 acétone 1), 19,5 g du composé 3 sous forme de diacétate et 2,5 g de l'azido-3 didésoxy-2,3 β-D-arabino-hexopyrannoside de méthyle sous forme de diacétate.

Ce dernier composé est repris dans 45 ml de méthanol anhydre puis on ajoute 5 ml de méthanolate de sodium. Après 4 heures d'agitation, la solution est neutralisée par addition de résine amberlite IRC50. Le filtrat évaporé donne l'azido-3 didesoxy-2,3 β-D-arabino-hexapyrannoside de méthyle sous forme de cristaux.
Analyse : $C_7H_{13}N_3O_4$ : 203,2

b) Préparation de l'amino-3 didesoxy-2,3 β-D-arabino-hexapyrannoside de méthyle

Ce composé est préparé en placant une solution de 5 g d'azido-3 didésoxy-2,3 β-D-arabino-hexopyrannoside de méthyle dans 10 ml de méthanol, et en agitant 12 heures sous atmosphère d'hydrogène en présence de triéthylamine (1 ml) et de charbon palladié à 10 % (1 g). Le catalyseur est éliminé par filtration.

Analyse : $C_7H_{15}NO_4$ : 177
F : 140°-142°
$[\alpha]_D$ : - 61,8° (c 0,55 % MeOH)

c) Préparation du [(chloro-2 éthyl)-3 nitroso-3 ureido]-3 didésoxy-2,3 β-D-arabino-hexopyrannoside de méthyle

L'amino-3 didesoxy-2,3 β-D-arabino-hexopyrannoside de méthyle est mis en solution dans du DMF (diméthyl formamide), puis on ajoute du N-(chloro-2-éthyl)-N-nitroso-carbamate de trichloro-2,4,5 phényle, selon le procédé de l'invention.

Après 4 heures d'agitation à 0°C, le milieu réactionnel est évaporé. Le composé obtenu (IC 85-1673) est un produit nouveau est ses caractéristiques sont les suivantes :

Analyse : $C_{10}H_{18}ClN_3O_6$ : 311,72 Rdt. : 70°
F : 68°-70°
$[\alpha]_D$ : - 37,9° (c 0,36 % MeOH)

Exemple 5 :

Préparation du [(chloro-2 éthyl)-3 nitroso-3 ureido]-3 tridesoxy-3,4,6 α-L-xylo-hexopyrannoside de méthyle (IC 1626)

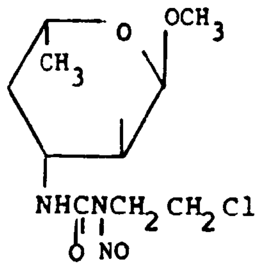

IC 1626

Ce composé est obtenu à l'aide de l'amino-3 tridésoxy-3,4,6 α-L-xylo-hexopyrannoside de méthyle préparé selon H. Baer, Canad. J. Chem., vol. 52, 1974, p.122-124, lequel est mis en solution dans du DMF, puis on ajoute du N-(chloro-2-éthyl)-N-nitroso-carbamate de trichloro-2,4,5 phényle, selon le procédé de l'invention.

Après 4 heures d'agitation à 0°C, le milieu réactionnel est évaporé. Le composé obtenu (IC. 1626) est un produit nouveau et ses caractéristiques sont les suivantes :

Analyse : $C_{10}H_{18}ClN_3O_5$ : 295,7 Rdt. : 65 %
F : 103°-105°
$[\alpha]_D$ = - 141,5 (c 0,71 $CHCl_3$)

Exemple 6 :

Préparation du [(chloro-2 éthyl)-3 nitroso-3 ureido]-3 tridésoxy-3,4,6 β-L xylo-hexopyrannoside de méthyle (IC 1627)

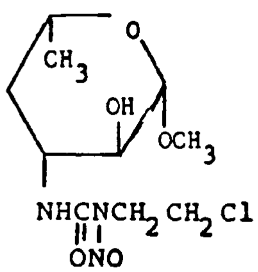

IC 1627

a) Préparation du nitro-3 tridésoxy-3,4,6 β-L xylo-hexopyrannoside de méthyle

A 1,8 g de l'anomère α-ci-dessus, on ajoute 40 ml de méthanol chlorhydrique N. Après deux heures au reflux, le milieu réactionnel est évaporé à sec puis purifié sur colonne de silice (éluant $CH_2Cl_2$-MeOH 98:2).

On obtient 500 mg de l'anomère β pur.

b) Préparation de l'amino-3 tridésoxy-3,4,6 β-L xylo-hexopyrannoside de méthyle

Le composé précédent est hydrogéné sous pression atmosphérique en présence d'oxyde de platine dans l'éthanol.

Analyse : $C_7H_{15}NO_3$ : 161 Rdt. : 80 %
F : 146°-148°
$[\alpha]_D$ = + 36,0 (c 0,96 $CHCl_3$)

c) Préparation du [(chloro-2 éthyl)-3 nitroso-3 uréido]-3 tridésoxy-3,4,6 β-L xylo hexopyrannoside de méthyle

L'amino-3 tridésoxy-3,4,6 β-L xylo-hexopyrannoside de méthyle est mis en solution dans du DMF, puis on ajoute du N-(chloro-2-éthyl)-N-nitroso-carbamate de trichloro-2,4,5 phényle, selon le procédé de l'invention.

Après 4 heures d'agitation à 0°C, le milieu réactionnel est évaporé. Le composé obtenu (IC. 1627) est un produit nouveau et ses caractéristiques sont les suivantes :

Analyse : $C_{10}H_8ClN_3O_5$ : 295,7
F : 90°-92° (éther isopropylique)
$[\alpha]_D$ : -9,0° (c 0,6 $CHCl_3$)

Exemple 7 :

Préparation du [(chloro-2 éthyl)-3 nitroso-3 ureido]-3 tridésoxy-3,4,6 α-D-xylo-hexopyrannoside de méthyle (IC 1590)

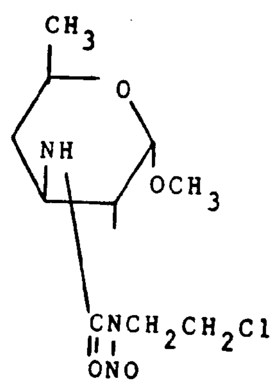

a) Préparation du O-acétyl-2 azido-3 dichloro 4-6 tridesoxy-3,4,6 α-D-galacto-hexopyrannoside de méthyle

A 44 g (0,21 mole) d'azido-3 déoxy-3 D glucopyrannose dans 160 ml de pyridine anhydre et 200 ml de chloroforme refroidis à - 78°C, sont ajoutés, goutte à goutte, 52 ml de chlorure de sulfuryle. Après deux heures à - 78°C, le mélange est agité pendant 5 heures à température ambiante.

Le milieu réactionnel est dilué avec 400 ml de chloroforme, puis lavé à l'acide chlorhydrique 2N, à l'eau, puis par une solution de bicarbonate de sodium et de nouveau avec de l'eau. Après séchage sur sulfate de sodium et évaporation à sec sous vide, le résidu est repris dans 200 ml de méthanol puis est ajoutée une solution à 10 % d'iodure de potassium dans un mélange eau-méthanol (1:1).

La solution neutralisée par du bicarbonate de potassium est filtrée, évaporée puis le résidu repris par du chloroforme. La phase organique, lavée par une solution de thiosulfate de sodium puis par de l'eau, est séchée sur sulfate de sodium puis évaporée.

Le résidu est repris dans 100 ml de pyridine refroidie à 0°. On ajoute goutte à 0° 20 ml d'anhydre acétique, on laisse sous agitation pendant une journée, puis le milieu réactionnel est évaporé sous vide, la phase aqueuse est extraite par 2 ou 3 fois 200 ml de chlorure de méthylène. La phase organique est ensuite lavée à l'eau, évaporée à sec, sous vide et on obtient 36 g d'un mélange correspondant aux deux anomères α et β.

Par chromatographie sur colonne de silice (éluant : hexane-acétone 10:1), on isole les deux anomères purs.

Anomère α 14,5 g F : 74°-76° (hexane-acétate d'éthyle)
$[\alpha]_D$ = + 171° (c, 1,17) $CHCl_3$)

Anomère β 16,0 g F : 108°-110° (hexane-acétate d'éthyle)
$[\alpha]_D$ = - 6,5° (c 1,58 $CHCl_3$)

b) Préparation de l'azido-3 dichloro-4,6 tridesoxy-3,4,6 α-D, galacto-hexopyrannoside de méthyle

5 g (0,017 mole) de l'anomère α précédent sont mis en solution dans 50 ml de méthanol anhydre en présence de 1 g d'acide tosylique. Le milieu réactionnel est laissé 18 heures à température ambiante puis évaporé à sec sous vide. Le résidu est repris par du chlorure de méthylène, la phase organique lavée 2 fois avec de l'eau est séchée, filtrée puis évaporée.
Les cristaux obtenus (4,2 g) sont recristallisés dans un mélange hexane-acétate d'éthyle.

Analyse : $C_7H_{11}Cl_2N_3O_3$ : 256
F : 139°-141°
$[\alpha]_D$ = + 188° (c 1,035 $CH_3OH$).

c) Préparation de l'amino-3 tridésoxy-3,4,6 α-D xylo-hexopyrannoside de méthyle

A 2 g (0,0075 mole) du composé précédent dans 50 ml de toluène anhydre sous azote sont ajoutés 0,5 g d'azo 2-2'bis isobutyronitrile puis, goutte à goutte, 8 ml d'hydrure de tributylétain. Le milieu réactionnel est porté au reflux pendant 10 heures. Après refroidissement et essorage du précipité, le filtrat est évaporé à sec sous vide. Par chromatographie sur silice (éluant $CH_2Cl_2$-MeOH ammoniacal 9:1), on obtient le sucre aminé pur sous forme de cristaux blancs.

Analyse : $C_7H_{15}NO_3$ : 161
F : 136°-139° (éther-méthanol)
$[\alpha]_D$ : +172° (c 1 % $CHTCl_3$)

d) Préparation du [(chloro-2 éthyl)-3 nitroso-3 uréido]-3 tridésoxy-3,4,6 α-D xylo-hexopyrannoside de méthyle (IC. 1591)

L'amino-3 tridésoxy-3,4,6 α-D xylo-hexopyrannoside de méthyle est mis dans du DMF, puis on ajoute du N-(chloro-2 ethyl)-N-nitroso-carbamate de trichloro-2, 4, 5 phényle, selon le procédé de l'invention.
Après 4 heures d'agitation à 0°C, le milieu réactionnel est évaporé. Le composé obtenu (IC 1590) est un produit nouveau et ses caractéritiques sont les suivantes :

Analyse : $C_{10}H_{18}ClN_3O_5$ : 295,7 Rdt. : 65 %
F : 103°-105°
$[\alpha]_D$ : +138° (c 1,41 $CHCl_3$)

Exemple 8 :

Préparation du [chloro-2 éthyl)-3 nitroso-3 ureido]-3 tridésoxy-3,4,6 β-D-xylo-hexopyrannoside de méthyle (IC. 1591)

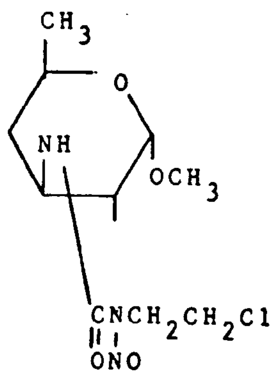

a) Préparation du O-acétyl-2 azido-3 dichloro-4,6 tridésoxy-3,4,6 β-D-galacto-hexopyrannoside de méthyle

A 44 g (0,21 mole) d'azido-3 déoxy-3 D glucopyrannose dans 160 ml de pyridine anhydre et 200 ml de chloroforme refroidis à -78° , sont ajoutés, goutte à goutte, 52 ml de chlorure de sulfuryle. Après deux heures à -78°C, le mélange est agité pendant cinq heures à température ambiante.

Le milieu réactionnel est dilué avec 400 ml, puis lavé à l'acide chlorhydrique 2N, à l'eau, puis par une solution de bicarbonate de sodium et de nouveau avec de l'eau. Après séchage sur sulfate de sodium et évaporation a sec sous vide, le résidu est repris dans 200 ml de méthanol puis est ajoutée une solution à 10 % d'iodure de potassium dans un mélange eau-méthanol (1 : 1).

La solution neutralisée par du bicarbonate de potassium est filtrée, évaporée puis le résidu repris par du chloroforme. La phase organique, lavée par une solution de thiosulfate de sodium puis par de l'eau; est séchée sur sulfate de sodium puis évaporée.

Le résidu est repris dans 100 ml de pyridine refroidie à 0°. On ajoute goutte à goutte à 0° 20 ml d'anhydre acétique, on laisse sous agitation pendant une journée, puis le milieu réactionnel est évaporé sous vide, la phase aqueuse est extraite par 2 ou 3 fois 200 ml de chlorure de méthylène. La phase organique est ensuite lavée à l'eau, évaporée à sec, sous vide et on obtient 36 g d'un mélange correspondant aux deux anomères α et β.

Par chromatographie sur colonne de silice (éluant hexane-acétone 10 :1), on isole les deux anomères purs.
Anomère α 14,5 g
F : 74°-76° (hexane-acétate d'éthyle)
$[\alpha]_D$ = +171° (c 1,17 $CHCl_3$)
Anomère β 16,0 g
F : 108°-110° (hexane-acétate d'éthyle)
$[\alpha]_D$ : -6,5° (c 1,58 $CHCl_3$)

b) Préparation de l'azido-3 dichloro-4,6 tridésoxy-3,4,6 β-D-galacto-hexopyrannoside de méthyle

On opère de la même manière que pour la partie b du IC 1590.

Analyse : $C_7H_{11}Cl_2N_3O_3$ : 256 Rdt. : 85 %
F : 135°
$[\alpha]_D$ : + 10,0° (c : 0,97 % $CH_3OH$)

c) Préparation de l'amino-3 tridésoxy-3,4,6 β-D-xylo-hexopyrannoside de méthyle

On procède de la même manière que pour la partie c du IC 1590.

Analyse : $C_7H_{15}NO_3$ : 161
F : 148°-149°
$[\alpha]_D$ : - 52° (c 1 % $CHCl_3$)

d) Préparation du [(chloro-2 éthyl)-3 nitroso-3 ureido]-3 tridésoxy-3,4,6 β-D-xylo-hexopyrannoside de méthyle

L'amino-3 tridésoxy-3,4,6 β-D-xylo-hexopyrannoside de méthyle est mis en solution dans du DMF, puis on ajoute du N-(chloro-2 éthyl)-N-nitroso-carbamate de trichloro 2, 4, 5 phényle, selon le procédé de l'invention.

Après 4 heures d'agitation à 0°C, le milieu réactionnel est évaporé. Le composé obtenu (IC 1591) est un produit nouveau et ses caractéristiques sont les suivantes :

Analyse : $C_{10}H_{18}ClN_3O_5$ : 295,7 Rdt. : 90 %
F : 84°-90°
$[\alpha]_D$ : + 11,5° (c 1,25 $CHCl_3$)

ETUDE DE L'ACTIVITE DES PRODUITS DES EXEMPLES 2 A 8 :

I - Matériel et méthodes

A- Tumeurs utilisées

Deux tumeurs murines ont été utilisées pour les études "in vivo" : la leucémie L1210 et le mélanome B16.

1) Leucémie L1210

. Animaux

Les expérimentations ont toujours été effectuées sur des souris femelles Indemnes d'Organismes Pathogènes Spécifiques (I.O.P.S.),
– soit de lignée DBA/2JIco
– soit B6 D2 F1/JIco (hybrides de première génération entre les lignées C57BL/6 et DBA/2).
Pour chaque expérimentation, la lignée est précisée.

. Greffe tumorale

Le jour de la greffe (par convention Jour 0 = Jo) chaque souris reçoit par voie intrapéritonéale (I.P.) un inoculum de $1x10^5$ cellules tumorales sous un volume de 0,2 ml.

Cet inoculum est préparé par dilution du liquide ascitique prélevé dans le péritoine d'une souris donneuse dans du milieu NCTC 109 (Laboratoire Eurobio Paris, France), numération au microscope dans une cellule de MALASSEZ et ajustement, à l'aide du même milieu, à la concentration de $5x10^5$ cellules par ml.

La lignée tumorale est la lignée L1210 de souche américaine.

. Répartition en séries expérimentales

Après la greffe tumorale, les souris sont réparties au hasard en cages de 5 animaux. Ensuite, ces cages de 5 souris sont elles-mêmes réparties par tirage au sort, en série témoin (contrôle) et en série(s) traitée(s) par le composé IC 1625 ou IC 1673 définis ci-dessus conformément au procédé de l'invention.

2) Mélanome B16

. Animaux

Les expérimentations ont toujours été effectuées sur des souris I.O.P.S. femelles, B6 D2 F1/JIco (hybrides

de première génération entre les lignées C57BL/6 et DBA/2).

. Greffe tumorale

Le jour de la greffe (Jour 0) chaque souris reçoit par voie intrapéritonéale (I.P.) un inoculum de $2x10^6$ cellules tumorales sous un volume de 0,5 ml. Cet inoculum est préparé à partir d'une tumeur sous-cutanée prélevée sur une souris donneuse. La tumeur prélevée est dilacérée à l'aide de ciseaux dans du milieu NCTC 109. Après filtration sur gaze stérile pour éliminer les fragments mal dilacérés, la suspension cellulaire homogène obtenue est numérée à l'aide d'une cellule de MALASSEZ, et ramenée à la concentration voulue ($4x10^6$ cellules tumorales par ml) par dilution dans du milieu NCTC 109.

. Répartition en séries expérimentales

Elle s'effectue comme pour la leucémie I1210 (voir plus haut).

b- protocole de traitement

les doses des produits IC 1625 ou IC 1673, utilisées lors des différentes expérimentations sont exprimées en milligrammes par kilogramme de poids corporel.

Les produits sont mis en solution dans le soluté injectable de chlorure de sodium isotonique.

Deux protocoles de traitement ont été utilisés lors des différentes expérimentations :

– soit une injection unique à J1, par voie intrapéritonéale (I.P.) ou intraveineuse (I.V.),
– soit trois injections I.P. à J1, J5, J9.

Dans chaque expérimentation, les animaux de la série témoin reçoivent suivant le protocole expérimental, une ou plusieurs injections, par la même voie (I.P. ou I.V.) d'un même volume (0,2 ml/20g) du véhicule sans principe actif (soluté isotonique de chlorure de sodium).

II- Expression des résultats

Pour chaque expérimentation un tableau précise :

– le nombre de souris survivantes à J60 avec le pourcentage correspondant,
– pour les séries traitées le T/C.
  . T représentant le temps médian de survie des souris de la série traitée,
  . C représentant le temps médian de survie des souris de la série contrôle (témoins).

III- Remarque

Les lignées de souris utilisées, les protocoles expérimentaux et le mode d'expression des résultats sont en conformité avec l'instruction 271 F de la "N.C.I. Division of Cancer Treatment" (november 1983).

IV- Résultats pharmacologiques

IV- Résultats pharmacologiques

IC. 1625

L1210 USA - $DBA_2$ - Traitement I.P. à JI, J5, J9

3 x 10 mg/kg

| T/C | Nombre de survivants à J60 |
|---|---|
| 190 | (0/10) |

IC. 1673

L1210 USA - $B_6D_2F_1$ - Traitement I.P. à J1, J5, J9
3 x 1,25 mg/kg

| T/C | Nombre de survivants |
|---|---|
| 158 | (0/10) |

L1210 USA - $B_6D_2F_1$ - Traitement I.P. à J1, J5, J9
3 x 5 mg/kg

| T/C | Nombre de survivants |
|---|---|
| 626 | (6/10) |

Mélanome B16 - $B_6D_2F_1$ - Traitement I.P. à J1, J5, J9
3 x 10 mg/kg

| T/C | Nombre de survivants |
|---|---|
| 232 | (4/10) |

IC 1626

L1210 USA - $DBA_2$ - Traitement I.P. à J1,
1 x 20 mg/kg,
Nombre de survivants à J60 : 3/10

L1210 USA - $DBA_2$ - Traitement I.P. à J1, J5, J9
3 x 2,5 mg/kg, 3 x 10 mg/kg

| | T/C | Nombre de survivants à J60 |
|---|---|---|
| 3x2,5 | 150 | 0/10 |
| 3x10 | 275 | 3/10 |

IC 1627

L1210 USA - $DBA_2$ - Traitement I.P. à J1,
1 x 20 mg/kg

Nombre de survivants à J60 : 3/10

L1210 USA - $DBA_2$ - Traitement I.P. à J1, J5, J9
3 x 2,5 mg/kg, 3 x 10 mg/kg

| | T/C | Nombre de survivants |
|---|---|---|
| 3x2,5 | 141 | 0/10 |
| 3x10 | >600 | 6/10 |

IC. 1591

L1210 souche USA - $DBA_2$ - Traitement I.P. à J1
1 x 20 mg/kg

| T/C | Nombre de survivants à J60 |
|---|---|
| >600 | 7/10 |

L1210 souche USA - $DBA_2$ - Traitement I.P. à J1, J5, J9
3 x 5 mg/kg

| T/C | Nombre de survivants à J60 |
|---|---|
| >600 | 6/10 |

L1210 souche USA - $B_6D_2F_1$ - Traitement I.P. à J1, J5, J9
3 x 1,25 mg, 3 x 5 mg,
3 x 10 mg/kg

| | T/C | Nombre de survivants à J60 |
|---|---|---|
| 3x1,25 | 116 | 0/10 |
| 3 x 5 | 217 | 2/10 |
| 3 x 10 | >600 | 8/10 |

Mélanome B16 - $B_6D_2F_1$ - Traitement I.P. à J1, J5, J9
3 x 10 mg/kg

| T/C | Nombre de survivants à J60 |
|---|---|
| 194 | 3/10 |

V- Résultats toxicologiques

DL0, DL50, Monodose I.P. sur des souris de lignées précédentes.
Pour le composé IC 1625, la DL0 est supérieure ou égale à 40 mg/kg.
Pour le composé IC 1673, la DL0 est voisine de 20 mg/kg.
Pour le composé IC. 1590 la DL0 est supérieure ou égale à 25 mg/kg.
Pour le composé IC. 1591 la DL0 est supérieure ou égale à 20 mg/kg.

## Revendications

1. Procédé de préparation des composés de formule I

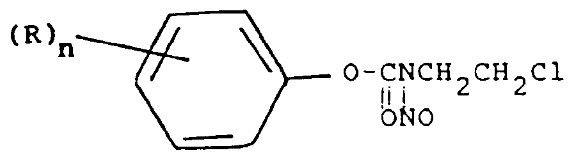

I

dans laquelle
n vaut 2 à 5 et R représente Cl, Br ou F caractérisé en ce que, dans une première étape, l'on fait réagir l'halogénoformiate de formule II

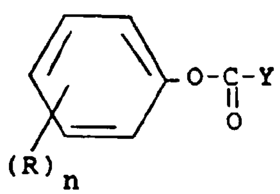

II

R et n ayant la signification indiquée ci-dessus, Y représentant un halogène, notamment Cl, sur $NH_2CH_2CH_2Cl$, pour obtenir le composé de formule Ibis

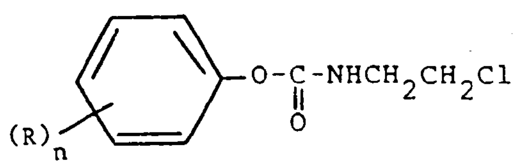

Ibis

dans laquelle R et n ont les significations indiquées ci-dessus, et en ce que, dans une deuxième étape, on traite directement le composé Ibis ci-dessus mentionné, par l'acide nitrosylsulfurique.

2. Procédé selon la revendications 1, caractérisé en ce que l'halogénoformiate est un chloroformiate choisi parmi le trichloro 2,4,5-phénylchloroformiate, le pentachlorophenylchloroformiate et le pentafluorophenylchloroformiate

3. Procédé selon la revendication 1, de préparation de composé de formule V :

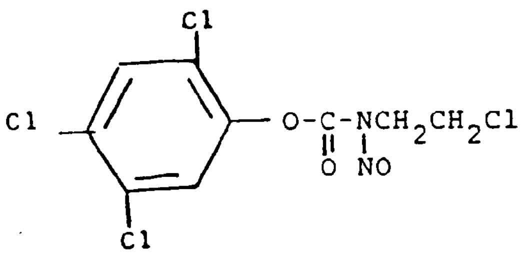

V

caractérisé en ce que, dans une première étape, l'on fait réagir le trichloro-2, 4, 5 phenylchloroformiate sur $ClCH_2CH_2NH_2$ pour obtenir le composé de formule IV :

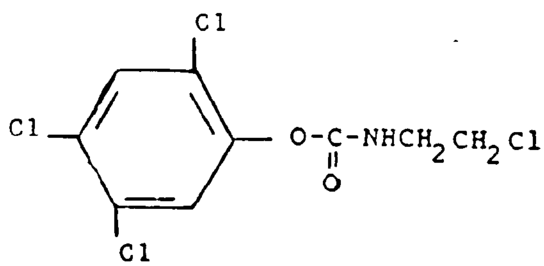

IV

et en ce que, dans une deuxième étape, l'on traite le produit ainsi obtenu directement avec de l'acide nitrosylsulfurique.

4. Procédé selon l'une quelconque des revendications 1 à 3, caractérisé en ce que l'on effectue la première étape dans du chlorure de méthylène anhydre.

5. Procédé selon l'une quelconque des revendications 1 à 4, caractérisé en ce que l'on effectue la deuxième étape dans de l'acide acétique anhydre.

6. Procédé de préparation de nitrosourée, caractérisée en ce que l'on met en oeuvre le procédé des revendications 1 à 5 pour obtenir un nitrosocarbamate de formule I :

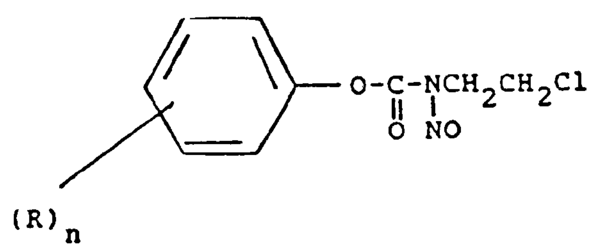

(I)

dans laquelle :
R et n ont les significations indiquées aux revendications 1 à 3, et en ce que l'on condense ce nitrosocarbamate sur l'amine d'un sucre à partir duquel on veut former la nitrosourée correspondante.

**Patentansprüche**

1. Verfahren zur Herstellung von Verbindungen der Formel I

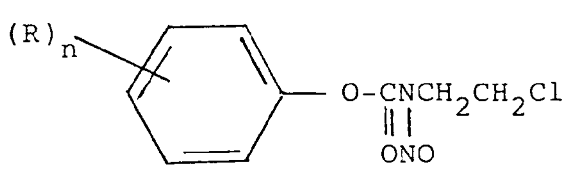

I ,

in der
n 2 bis 5 und R Cl, Br oder F bedeuten, **dadurch gekennzeichnet**, daß man in einer ersten Stufe ein Halogenformiat der Formel II,

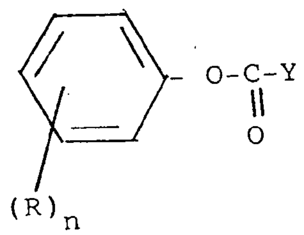

II,

in der R und n die oben angegebene Bedeutung haben und Y ein Halogen, insbesondere Chlor, darstellt, mit $NH_2CH_2CH_2Cl$ zu Verbindungen der Formel Ibis

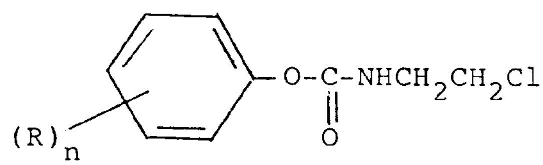

Ibis

umsetzt, in der R und n die oben angegebene Bedeutung haben, und in einer zweiten Stufe direkt die Verbindung Ibis mit Nitrosylschwefelsäure behandelt.

2. Verfahren nach Anspruch 1, dadurch gekennzeichnet, daß das Halogenformiat ein Chlorformiat ist, das unter 2,4,5-Trichlorphenylchlorformiat, Pentachlorphenylchlorformiat und Pentafluorphenylchlorformiat ausgewählt ist.

3. Verfahren nach Anspruch 1 zur Herstellung der Verbindung der Formel V,

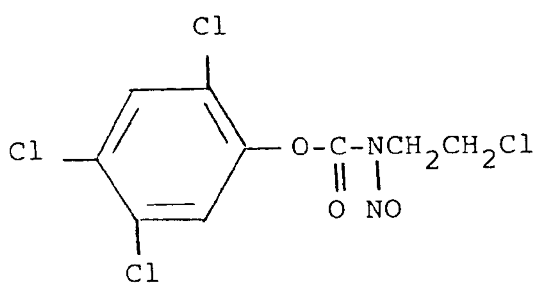

V,

dadurch gekennzeichnet, daß man in einer ersten Stufe 2,4,5-Trichlorphenylchlorformiat mit $ClCH_2CH_2NH_2$ zu der Verbindung der Formel IV

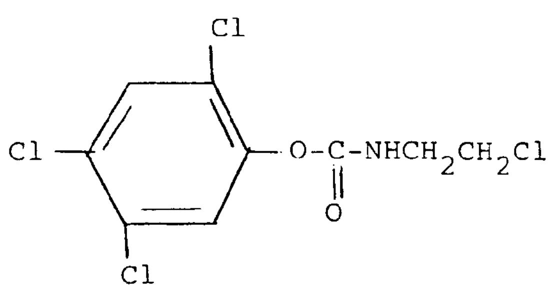

IV

umsetzt und in einer zweiten Stufe das so erhaltene Produkt direkt mit Nitrosylschwefelsäure behandelt.

4. Verfahren nach einem der Ansprüche 1 bis 3, dadurch gekennzeichnet, daß man die erste Stufe in wasserfreiem Methylenchlorid durchführt.

5. Verfahren nach einem der Ansprüche 1 bis 4, dadurch gekennzeichnet, daß man die zweite Stufe in wasserfreier Essigsäure durchführt.

6. Verfahren zur Herstellung von Nitrosoharnstoffen, dadurch gekennzeichnet, daß man das Verfahren der Ansprüche 1 bis 5 zur Herstellung eines Nitrosocarbamats der Formel I durchführt,

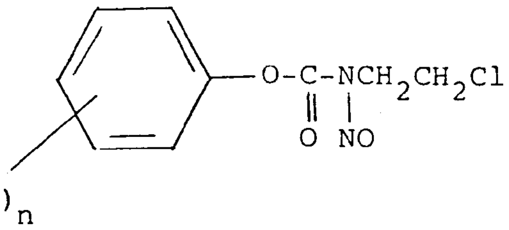

(I),

worin
R und n die in den Ansprüchen 1 bis 3 angegebene Bedeutung besitzen, und dieses Nitrosocarbamat mit einem Aminozucker kondensiert, von dem ausgehend der entsprechende Nitrosoharnstoff hergestellt werden soll.

## Claims

1. Process for preparing compounds of formula I

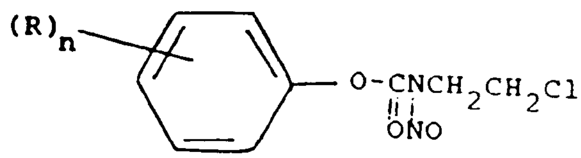

I

wherein
n represents 2 to 5 and R represents Cl, Br or F, characterised in that, in a first step, the haloformate of formula II

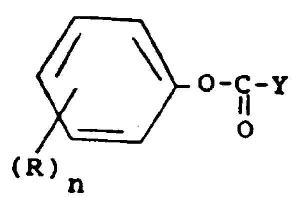

II

wherein R and n are as hereinbefore defined, and Y represents a halogen, particularly Cl, is reacted with $NH_2CH_2CH_2Cl$ to obtain the compound of formula I bis

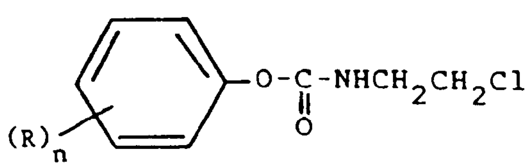

Ibis

wherein R and n are as hereinbefore defined, and, in a second step, the compound Ibis mentioned hereinbefore is treated directly with nitrosylsulphuric acid.

2. Process according to claim 1, characterised in that the haloformate is a chloroformate selected from among 2,4,5-trichloro-phenyl chloroformate, pentachlorophenyl chloroformate and pentafluorophenyl

chloroformate.

3. Process according to claim 1 for preparing compounds of formula V

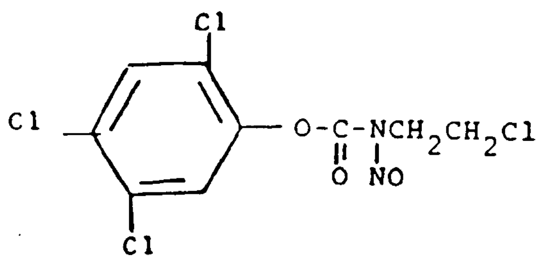

V

characterised in that, in a first step, the 2,4,5-trichloro-phenyl chloroformate is reacted with $ClCH_2CH_2NH_2$ to obtain the compound of formula IV

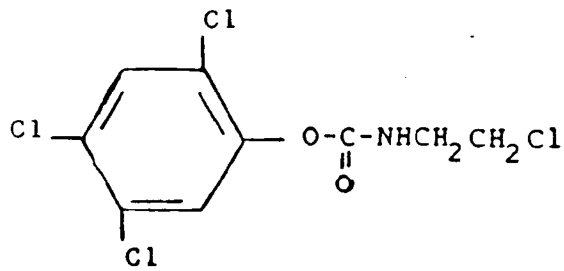

IV

and, in a second step, the product thus obtained is treated directly with nitrosylsulphuric acid.

4. Process according to any one of claims 1 to 3, characterised in that the first step is carried out in anhydrous methylene chloride.

5. Process according to any one of claims 1 to 4, characterised in that the second step is carried out in anhydrous acetic acid.

6. Process for preparing nitrosourea, characterised in that the process according to claims 1 to 5 is carried out in order to obtain a nitrosocarbamate of formula I

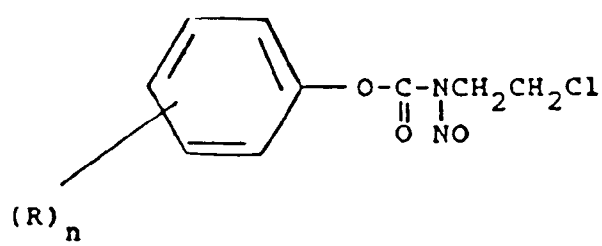

(I)

wherein

R and n have the meanings given in claims 1 to 3, and this nitrosocarbamate is condensed with the amine of a sugar from which it is desired to form the corresponding nitrosourea.

CARBAMATE DU TRICHLOROPHENOL / CHLOROFORME-D

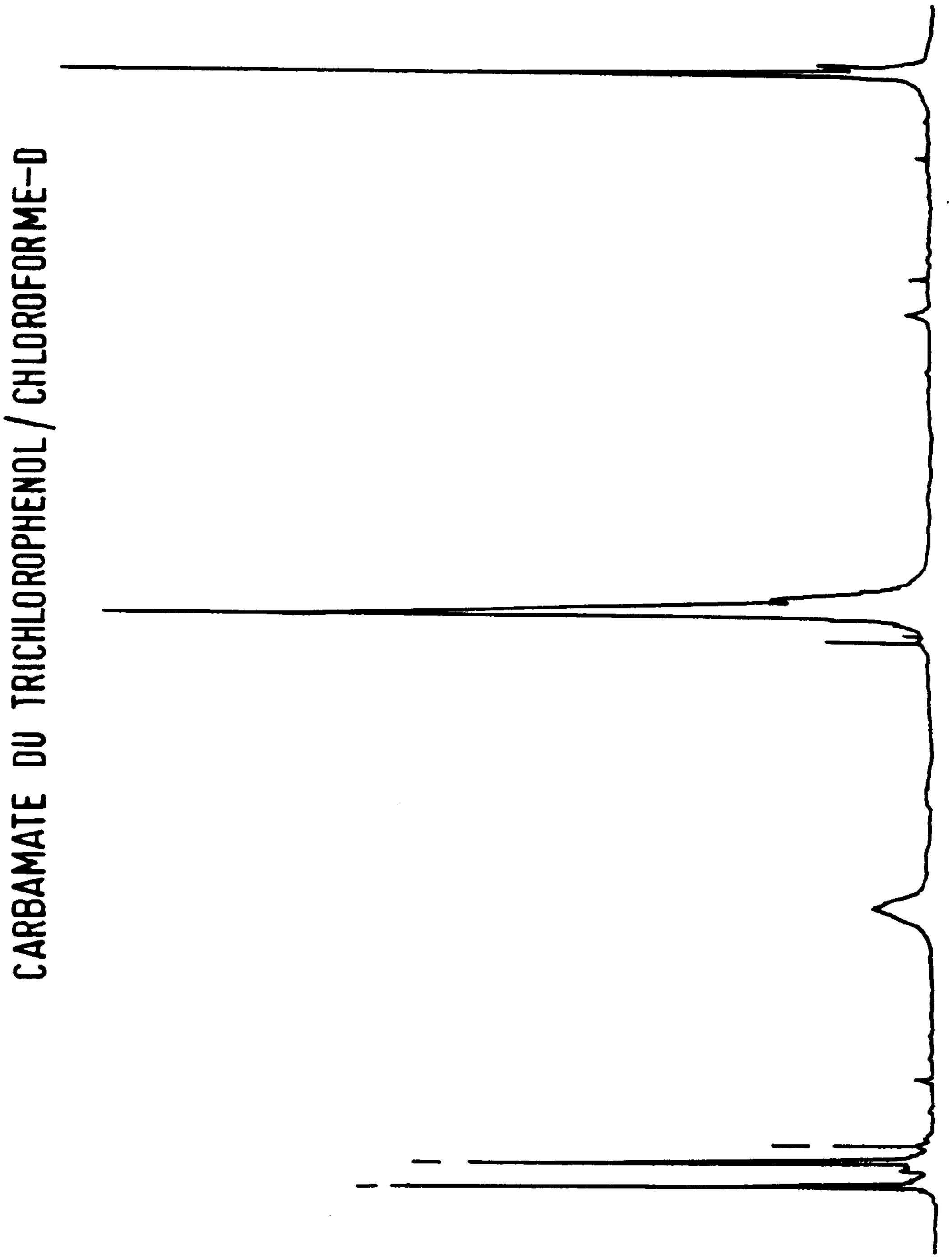

SPECTRE RMN (100MHz)

FIG.1

10-95-/CHLOROFORME-D

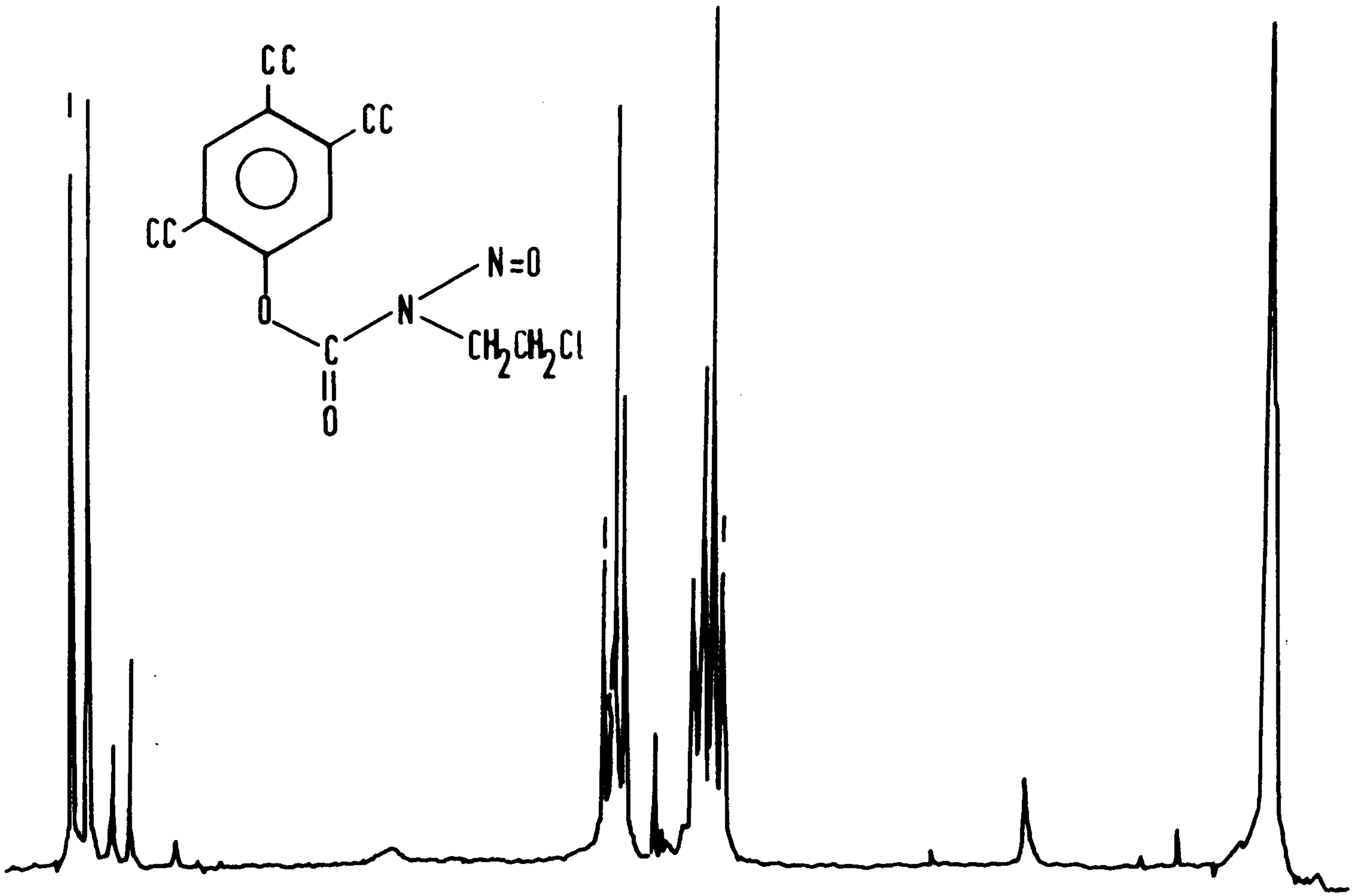

SPECTRE RMN (100MHz)

FIG. 2